# EUROPEAN PATENT SPECIFICATION

(11) **EP 0 685 557 B1**
(45) Date of publication and mention of the grant of the patent: **12.08.1998**
(21) Application number: 95902274.0
(22) Date of filing: 30.11.1994
(51) Int. Cl.: C12N 15/57, C12N 9/64

(54) **NOVEL METALLOPROTEASE AND DNA CODING FOR THE SAME**
NEUE METALLOPROTEASE UND KODIERENDE DNA DAFÜR
NOUVELLE METALLOPROTEASE ET ADN CODANT POUR CETTE DERNIERE

(30) Priority: 30.11.1993 JP 341061/93
(43) Date of publication of application: 06.12.1995
(73) Proprietor: FUJI YAKUHIN KOGYO KABUSHIKI KAISHA, Takaoka-shi Toyama 933 (JP)
(72) Inventor: SEIKI, Motoharu, Ishikawa 920 (JP); SATO, Hiroshi, Kanazawa-shi, Ishikawa 921 (JP); SHINAGAWA, Akira, 530, Chokeiji, Takaoka-shi, Toyama 933 (JP)
(74) Representative: Vossius, Volker, Dr.
(86) International application number: JP9402009
(87) International publication number: WO9515374

(56) References cited:
- WO-A-92/09701
- Nature, Vol. 370, No. 6484, July 7, 1994 (07-07-94), HIROSHI SATO et al., "A Matrix Metalloproteinase Expressed on the Surface of Invasive Tumour Cells", p. 61-65.
- Journal of Cancer Research Clinical Oncology, Vol. 117, No. 2, (1991), M. SIADAT PAJOUR et al., "Expression of Metalloproteinase Genes in Human Prostate Cancer", p. 144-150.

## Description

### TECHNICAL FIELD

The present invention relates to a novel metalloproteinase useful in applications such as diagnosis of the presence of tumour cells, diagnosis of the degree of tumour malignancy, or other medical or physiological fields.

More specifically, the present invention relates to one type of metalloproteinase expressed specifically in human tumour cells and a DNA sequence encoding therefor; a plasmid having a nucleotide sequence which contains said DNA sequence; a host cell harbouring said plasmid; a method for manufacturing said protein using said host cell; a probe which hybridizes with the aforesaid DNA sequence; a method for detecting DNA or RNA containing the aforesaid sequence using said probe; and monoclonal antibodies which bind specifically to the aforesaid protein.

### BACKGROUND

A group of enzymes with different substrate specificity and referred to in general as matrix metalloproteinases (hereinafter referred to as "MMPs") contributes to degradation of the extracellular matrix comprising such complex components as collagen, proteoglycan, elastin, fibronectin, and laminin.

Previously reported MMPs include interstitial collagenase (MMP-1), 72 kDa gelatinase (also known as type IV collagenase or gelatinase A; MMP-2), 92 kDa gelatinase (also known as type IV collagenase or gelatinase B; MMP-9), stromelysin-1 (MMP-3), matrilysin (MMP-7), neutrophil collagenase (MMP-8), stromelysin-2 (MMP-10) and stromelysin-3 (MMP-11).

These MMPs are a family of enzymes whose primary structure has been reported previously. With the exception of MMP-7, the primary structure among the family of reported MMPs comprises essentially an N-terminal propeptide domain, a Zn²⁺ binding catalytic domain and a C-terminal hemopexin-like domain. In MMP-7 there is no hemopexin-like domain. MMP-2 and MMP-9 contain an additional gelatin-binding domain. In addition, a proline-rich domain highly homologous to a type V collagen α2 chain is inserted in MMP-9 between the Zn²⁺ binding catalytic domain and the C-terminal hemopexin-like domain.

In highly metastatic tumour cells, there are reports of conspicuous expression of type IV collagenase (MMP-2, MMP-9) which mainly degrade type IV collagen (Cancer Res., 46:1-7, 1986; Biochem. Biophys. Res. Commun., 154:832-838, 1988; Cancer, 71:1368-1383, 1993). Likewise, it has been reported MMP-3 act as an activator of proMMP-9 (J. Biol. Chem., 267:3581-3584, 1992).

The degree of matrix metalloproteinase expression serves as an index to diagnosing the degree of cancer malignancy.

### DISCLOSURE OF THE INVENTION

The present inventors discovered a novel matrix metalloproteinase (hereinafter referred to as "MT-MMP") and performed a structural analysis thereof.

As described hereafter, the present invention offers a novel metalloproteinase protein, DNA having a nucleotide sequence which encodes said protein, a plasmid having said DNA nucleotide sequence, a host cell harbouring said plasmid and monoclonal antibodies which specifically recognize the aforesaid metalloproteinase protein.

One aspect of the present invention refers to a human matrix metalloproteinase having a length of 582 amino acids, having the amino acid sequences SEQ ID NO:3, SEQ ID NO:8, SEQ ID NO:9 and SEQ ID NO:10 at amino acid numbers 91-97, 160-173, 320-333 and 498-512, respectively, and having the sequence of hydrophobic amino acids shown in SEQ ID NO:7 at amino acid number 533-562. A further aspect of invention refers to a DNA encoding the human matrix metalloproteinase of the present invention. A further aspect of invention refers to an expression plasmid containing a DNA encoding the human matrix metalloproteinase of the present invention. A further aspect of invention refers to a host cell harboring the expression plasmid of the present invention. A further aspect of invention refers to monoclonal antibodies which recognize the human matrix metalloproteinase of the present invention. A further aspect of invention refers to the use of the DNA or the monoclonal antibodies of the present invention for the preparation of a composition for the diagnosis of the presence of tumor cells or the degree of tumor malignancy.

The present invention is described in detail hereafter.

Using highly conserved sequences (Sequence Sheet sequence numbers 3 and 4) selected from amino acid sequences of the known matrix metalloproteinase (MMP) family, the present inventors designed and synthesized an oligonucleotide primer having the sequences denoted by Sequence Sheet sequence numbers 5 and 6. A PCR was carried out using said oligonucleotide primer and a human placental cDNA library, the PCR products obtained were sequenced, and a 390 bp DNA fragment having a sequence non-homologous to known MMP was obtained. Using this 390 bp DNA fragment as a probe, the human placenta cDNA library was screened, and a cDNA in the positive phage clone obtained was sequenced. The nucleotide sequence is that denoted by Sequence Sheet sequence number 2. A sequence identical to the nucleotide sequence in Sequence Sheet sequence number 2 did not exist in the Genbank/EMBL DNA database, and DNA having this nucleotide sequence was ascertained to be completely novel.

The nucleotide sequence of the aforesaid cloned cDNA in Sequence Sheet sequence number 2 had a 3' non-coding sequence and an open reading frame that potentially encode 582 amino acids. An initiation codon was located at nucleotide number 112, and a stop codon was present at nucleotide number 1858. It was determined that this open reading frame encoded the 582 amino acid sequence in Sequence Sheet sequence number 1, that a deduced signal sequence continued after the initiation codon, and that a hydrophobic domain (Sequence Sheet sequence number 7) specific to a membrane-binding protein of 20 or more linked hydrophobic amino acids was present from C-terminal amino acid number 533 to 562.

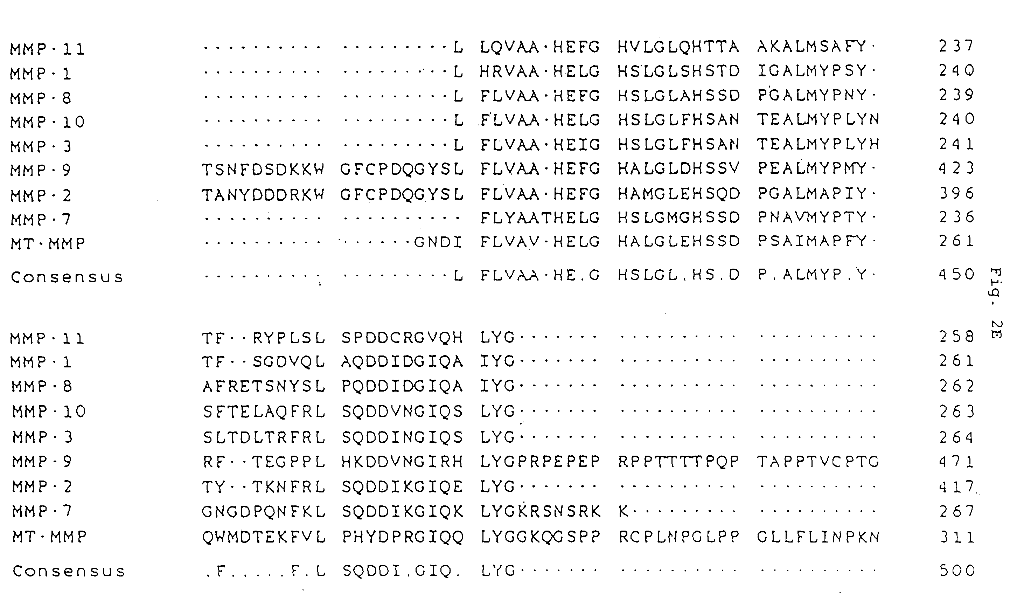

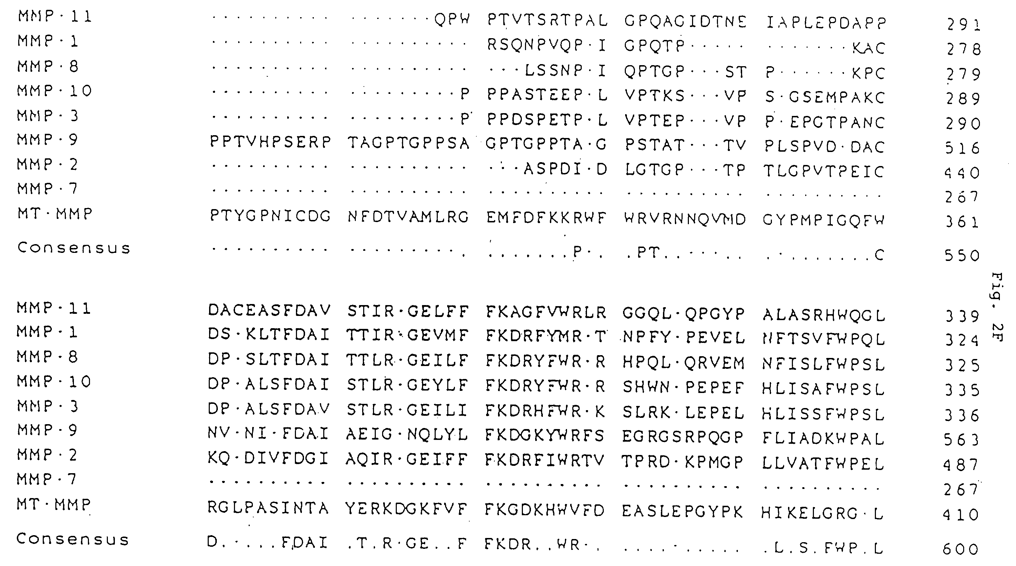

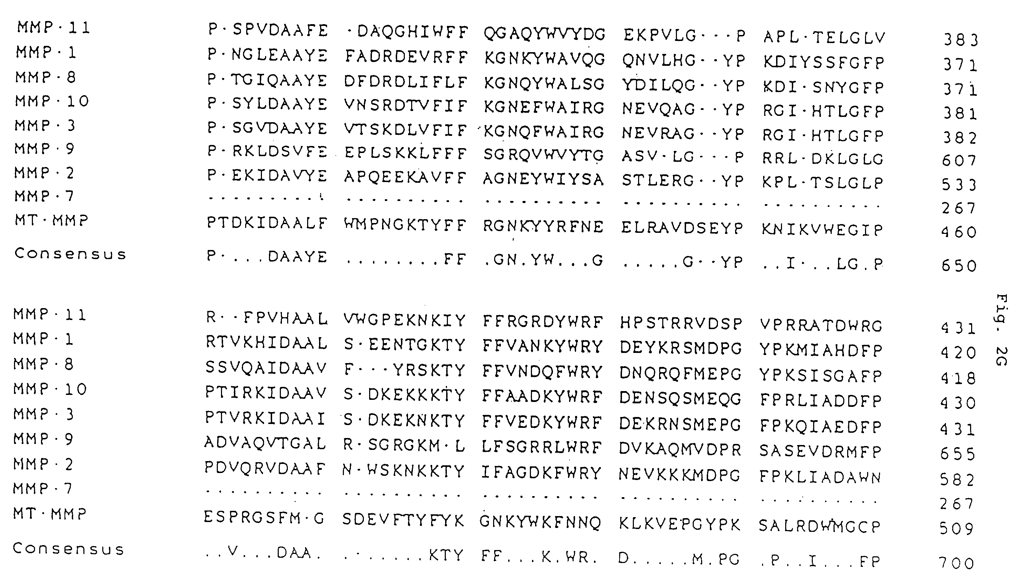

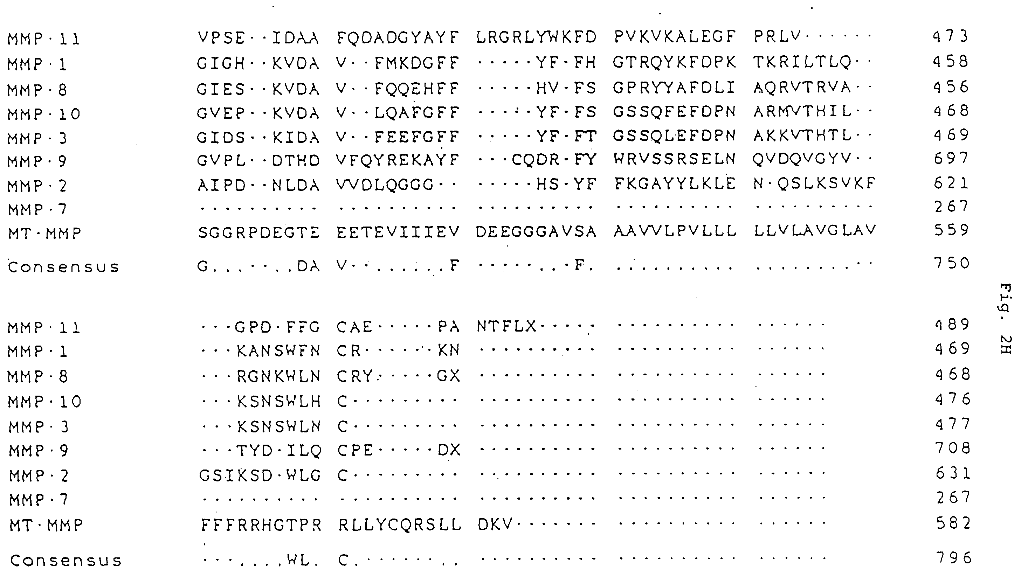

When homology between the amino acid sequence of MT-MMP and that of the known MMP family was analyzed, MT-MMP had high homology to the known MMP family, as shown in Figure 2. The sequences best conserved in MT-MMP were active site sequences, as well as sequences proximal to processing site between precursor and mature substance conserved in the MMP family. The fact that MT-MMP has the structural characteristics of a membrane-binding protein, and the presence in MT-MMP of a sequence of linked hydrophobic amino acids (shown in Sequence Sheet sequence number 7) not found in the rest of the MMP family, strongly suggested that MT-MMP, unlike other MMP family, is a membrane-binding MMP.

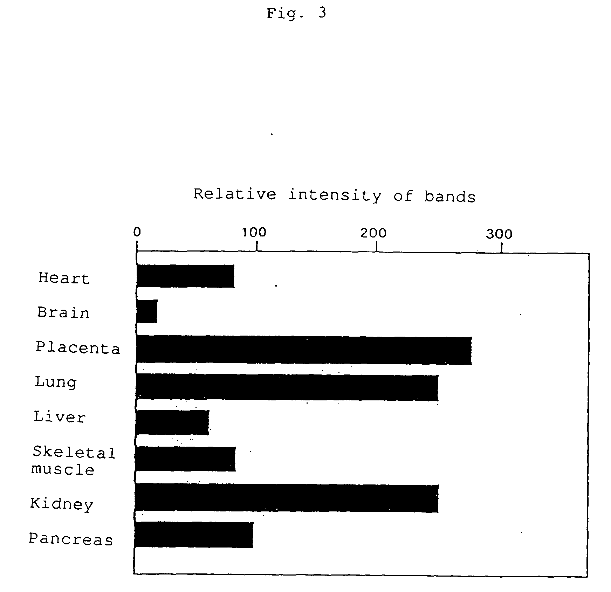

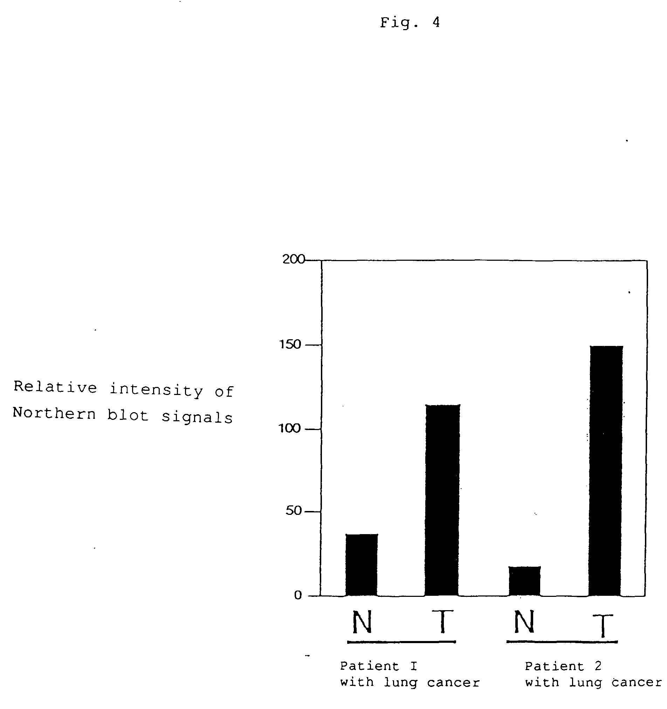

When MT-MMP expression in various human tissues was studied by Northern Blot analysis with various tissue-derived poly(A)⁺RNA, high expression was seen in the placenta, lung and kidney (see Figure 3). Likewise, results from Northern Blot analysis performed with RNA extracted from normal and tumour areas of human lung squamous cell carcinoma showed that MT-MMP is expressed peculiarly at tumour sites (see Figure 4).

Finally, immunoprecipitation and immunostain experiments using anti-MT-MMP monoclonal antibodies showed that the MT-MMP pertaining to the present invention is expressed on a cell membrane without secretion of a gene product, and MMP-2 activation induced by the expression of MT-MMP was observed in the cells transfected with MT-MMP gene (Nature, 370:61-65, 1994).

Due to the achievements of the above-discussed research by the present inventors, the present invention offers a novel matrix metalloproteinase protein having a length of 582 amino acids, having the amino acid sequences SEQ ID NO:3, SEQ ID NO:8, SEQ ID NO:9 and SEQ ID NO:10 at amino acid numbers 91-97, 160-173, 320-333 and 498-512, respectively, and having the sequence of hydrophobic amino acids shown in SEQ ID NO:7 at amino acid number 533-562.

In addition, the present invention offers DNA encoding said matrix metalloproteinase; a plasmid containing and capable of expressing said DNA; and a host cell harbouring said plasmid. All host cells used in general recombinant DNA technology can be used as the aforementioned host cell, including prokaryotes such as E. coli and Bacillus subtilis; eukaryotes such as yeast, COS cells, CHO cells and 3T3 cells; and insect cells such as Sf21. Expression vectors corresponding to used host cells can be used as the aforementioned plasmid.

Furthermore, the present invention offers mRNA transcribed from DNA encoding said matrix metalloproteinase of the present invention.

The present invention also offers a probe which hybridizes with the aforementioned DNA or RNA and specifically detects said DNA or RNA, and said probe may be one having any part of the nucleotide sequence of the DNA encoding the matrix metalloproteinase of the present invention, provided said probe is labeled by a generally used radioactive isotope or enzyme or the like, hybridizes specifically with said DNA or RNA in general blotting analysis and *in situ* hybridization, and accomplishes detection.

Furthermore, the present invention offers monoclonal antibodies which bind peculiarly with the MT-MMP pertaining to the present invention.

The monoclonal antibodies pertaining to the present invention can be prepared by a well-known method such as the method of Milstein et al. (Nature, 256:495-497, 1975) using human MT-MMP as an antigen. In this method, the antigen may be native human MT-MMP, recombinant human MT-MMP, or a synthetic peptide having a partial amino acid sequence of either.

By means of the present invention, DNA having a nucleotide sequence which encodes a protein with the amino acid sequence of the novel MT-MMP pertaining to the present invention can be cloned, and such DNA and a protein encoded by such DNA can be prepared by a genetic engineering technique. Through the use of a cDNA clone of such a novel MT-MMP, techniques generally used in genetic engineering can be used to clone the aforementioned nucleotide sequence into another vector or host. Based on the aforementioned cDNA nucleotide sequence, DNA appropriately suited to a probe may be designed and prepared.

Use of the above-discussed various implementations of the present invention offers various technical means applicable to applications pertaining to diagnostic agents or diagnostic methods used for diagnosis of the presence of tumour cells or for diagnosis of the degree of tumour malignancy, as well as applications in other medical or physiological fields.

The present invention is described in detail hereafter by means of Working Examples, but the present invention is not limited by these Working Examples.

### WORKING EXAMPLES

### Working Example 1 Isolation of novel metalloproteinase (MT-MMP) cDNA

### (a) Construction of cDNA Library

Total RNA was extracted from human placenta tissue by a guanidine-cesium chloride method (Biochemistry, 18:5294-5299, 1979) and poly(A)⁺RNA was purified using an oligo(dT)-cellulose column. Using a purified poly(A)⁺RNA as a template and an oligo(dT) primer, cDNA was synthesized according to the Gubler-Hoffman method (Gene, 25:263-269, 1983). The ends of the cDNA were converted to blunt end with T₄ DNA polymerase, and EcoR I sites present in the cDNA were methylated by EcoR I methylase. Using T₄ DNA ligase, an EcoR I linker [d(pG-G-A-A-T-T-C-C)] and the cDNA were ligated, and cDNA possessing EcoR I sites at both ends was generated by EcoR I digestion. Using T₄ DNA ligase, this cDNA was cloned into EcoR I site of λgt11. *In vitro* packaging of this cDNA was carried out, for example, using an *in vitro* packaging kit (Amersham), and a cDNA library was thus constructed. A commercial cDNA library such as a human placenta cDNA library (Clontech) can be used as a cDNA library.

### (b) Preparation of synthetic oligonucleotide primer

The sequences denoted by Sequence Sheet sequence numbers 3 (P-1) and 4 (P-2) were selected from among amino acid sequences of the known MMP family as highly conserved amino acid sequences in the MMP family, and oligodeoxynucleotide primers corresponding respectively to oligopeptide P-1 and oligopeptide P-2 were designed. Specifically, when amino acids coded by two or more codons were present in an oligopeptide, the sequences were designed as a mixture as shown in Sequence Sheet sequence numbers 5 (primer 1) and 6 (primer 2). Primer 1 and primer 2 were synthesized by a β-cyanoethyl phosphoamidite method using a DNA synthesizer (Applied Biosystems, Model 392). Using a NICK column (Pharmacia) equilibrated with 10mM sodium phosphate buffer, pH 6.8 the obtained primer 1 and primer 2 were purified.

### (c) Gene amplification by PCR

Using a human placenta-derived cDNA as a template and primers 1 and 2 noted in the above section (b), a PCR (PCR Technology, Stockton Press, pp. 63-67, 1989) was run.

As a result, a 390 bp PCR product was yielded. The obtained PCR product was cloned in an appropriate plasmid, e.g., pUC 119 or pBluescript, and the nucleotide sequence of the PCR product was determined using a fluorescence DNA sequencer (Applied Biosystems, Model 373A) and a Taq dye-primer cycle sequencing kit (Applied Biosystems). Among various PCR products whose nucleotide sequences were determined, PCR product A having no homology to nucleotide sequences of previously reported MMPs was obtained. PCR product A was used as a probe for screening the human placenta cDNA library noted in the foregoing section (a). ³²P labeling of the probe was generated using a random primed DNA labeling kit (Boehringer Mannheim).

### (d) Screening of novel MMP gene from cDNA library and DNA sequencing.

Host E. coli Y1090 was transfected with the human placenta cDNA library constructed in the λgt11 cited in the foregoing section (a) and plaques were formed. Specifically, Y1090 was cultured overnight in an L broth containing 0.02% maltose, and bacteria were harvested and suspended in 10mM MgSO₄. This cell suspension and a phage solution were mixed, incubated at 37°C for 15 minutes, and then the phages were adsorbed onto the host bacteria. Soft agar was added thereto, and the material was spread on an L plate (the above-noted operation is hereinafter termed "plating"). The plate was incubated overnight at 42°C and a plaque was formed, after which a nylon filter (e.g., Hybond-N, Amersham) or a nitrocellulose filter (e.g., HATF, Millipore) was placed onto the plate and left in place for approximately 30 seconds. The filter was gently peeled and immersed in an alkaline denaturant (0.1M NaOH, 1.5M NaCl) for 30 seconds, then immersed in a neutralizing solution (0.5M Tris-HCl buffer, pH 8 containing 1.5M NaCl) for 5 minutes. The filter was then washed with 2x SSPE (0.36M NaCl, 20mM NaH₂PO₄, 2mM EDTA) and dried. The foregoing plaque-to-filter transfer was repeated, and at least two filters were prepared. However, plate contact time for the second and subsequent filters was extended to approximately 2 minutes. Filters were baked 2 hours at 80°C and DNA was thus fixed. The two filters, at a minimum, prepared from one plate were respectively washed 1 hour at 42°C in a wash solution (50mM Tris-HCl buffer, pH 8.0 containing 1M NaCl, 1mM EDTA and 0.1% SDS), placed in a hybridization bag, and prehybridization was carried out by 6 to 8 hours immersion at 42°C in a prehybridization solution [50% formamide, 5x Denhardt's solution (0.2% bovine serum albumin, 0.2% polyvinylpyrolidone), 5x SSPE, 0.1% SDS, 100µg/ml heat-denatured salmon sperm DNA]. Next, the ³²P-labeled probe noted in section (c), heat-denatured for 5 minutes at 100°C, was added to the prehybridization solution, and hybridization was carried out overnight at 42°C. After hybridization was complete, the filters were washed at room temperature with an excess of 2x SSC solution containing 0.1% SDS. Next, the filters were placed for 15 minutes at 68°C in 1x SSC solution containing 0.1% SDS. The filters were then dried, layered with X-ray film (Kodak XR), and 1 week autoradiography was then carried out at -70°C. The X-ray films were developed, replica filters in duplicate produced from one plate were piled up each other, and signals that appeared precisely same place on duplicate filters were marked. Plaques corresponding to marked signals were suspended in SM solution (50mM Tris-HCl buffer, pH 7.5 containing 0.1M NaCl and 10mM MgSO₄). These phage suspensions were appropriately diluted and plating was performed, screening similar to that noted above was carried out, and recombinant phages were obtained.

### (e) Preparation of recombinant λgt11 DNA

Each cloned phages was plated, incubated for 3 hours at 42°C, and incubated overnight at 37°C. Several drops of chloroform was then added to the SM solution and the material was left at room temperature for 30 minutes. The SM solution together with the upper layer of soft agar was then scraped off, and centrifuged. Polyethylene glycol was added to a 10% final concentration in the supernatant, and the material was mixed and left at 4°C for 1 hour. The material was then centrifuged, the supernatant was discarded, and phage particles were collected. The phage particles were suspended in SM solution and purified by a glycerol gradient ultracentrifugation method (see "Molecular Cloning, a Laboratory Manual", T. Maniastis et al., Cold Spring Harbor Laboratory Press pp. 2.78, 1989). The phages obtained were suspended in SM solution and treated with DNase I and RNase A. A mixture of 20mM EDTA, 50µg/ml proteinase K, and 0.5% SDS was then added, and the material was incubated at 65°C for 1 hour. The material was then subjected to phenol extraction and diethylether extraction, and DNA was precipitated by ethanol precipitation. The DNA obtained was washed with 70% ethanol, dried, and dissolved in TE solution (10mM Tris-HCl buffer, pH 8 containing 10mM EDTA).

### (f) Sequencing of the insertion fragment

The λgt11 DNA prepared in the above section (e) was digested with EcoR I, an insertion fragment was excised and purified, and cloned into the EcoR I site of a pBluescript (Stratagene) vector. E. coli NM522 XLI-Blue was transformed with this recombinant pBluescript. The F' transformed cells were selected, infected with helper phage VCSM13 (Stratagene), and cultured overnight. The culture was centrifuged and the bacteria were removed, and PEG/NaCl was added to precipitate the phages. The precipitate was suspended in TE solution, and single-stranded DNA was extracted with phenol and recovered by ethanol precipitation. The single-stranded DNA was sequenced using a fluorescence DNA sequencer (Applied Biosystems, Model 373A) and a Taq dye-primer cycle sequencing kit (Applied Biosystems). The total length of the sequence determined was 3403 base pairs, and the sequence thereof is denoted by Sequence Sheet sequence number 2. The nucleotide sequence in Sequence Sheet sequence number 2 was searched using the Genbank/EMBL DNA database, but an identical sequence did not exist.

### (g) Analysis of Gene Product

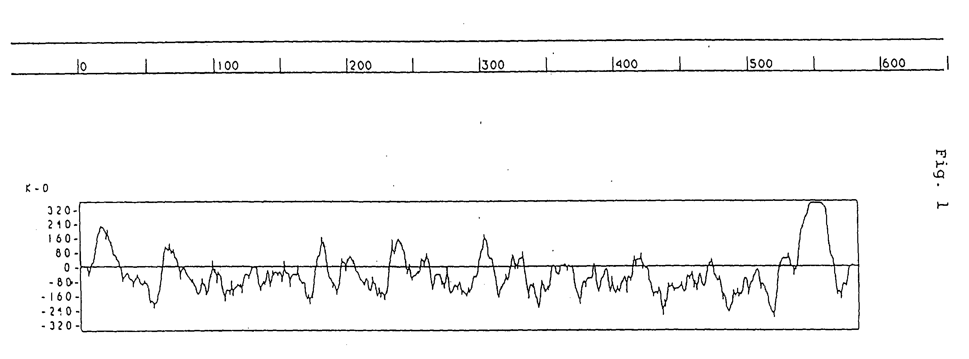

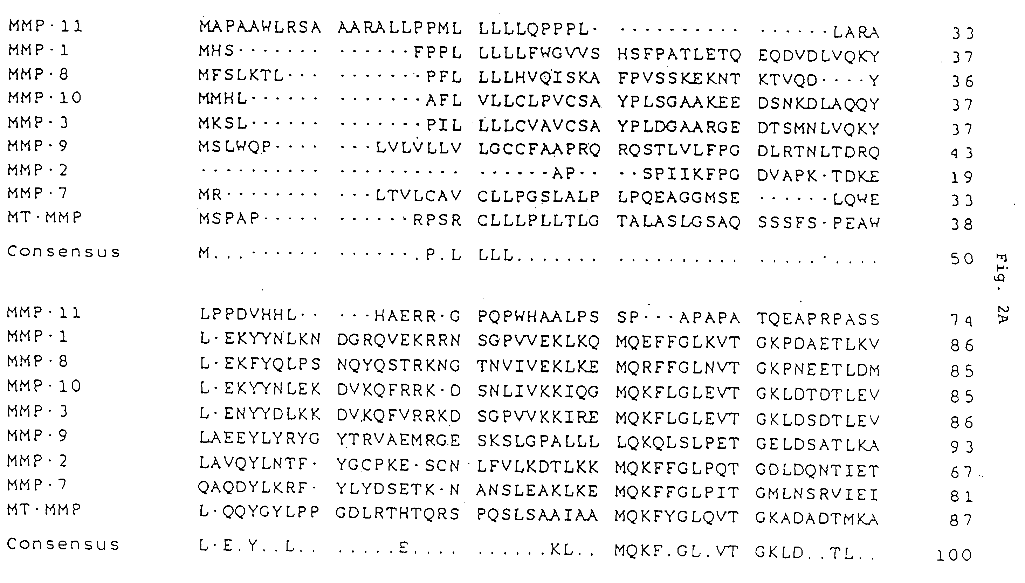

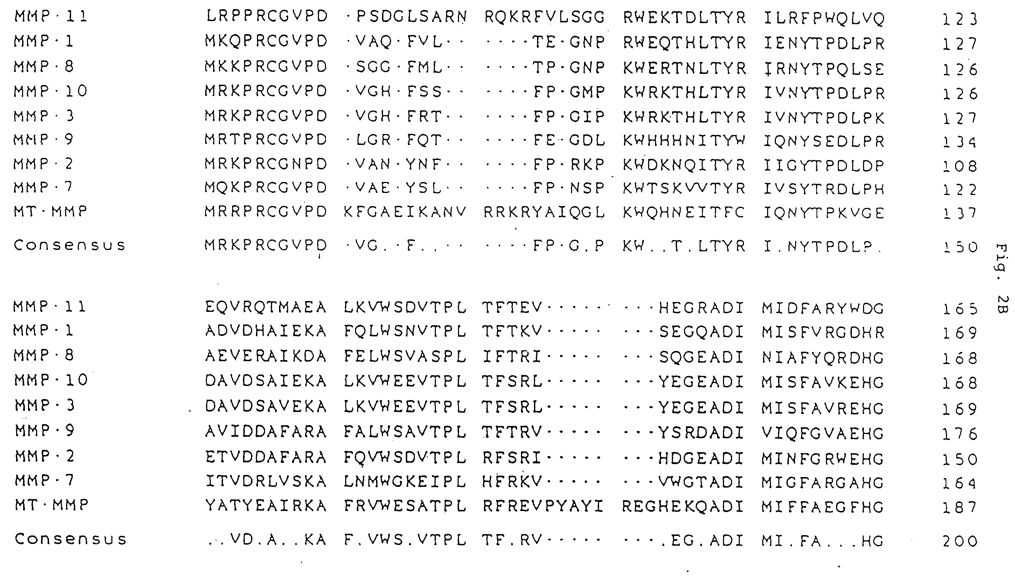

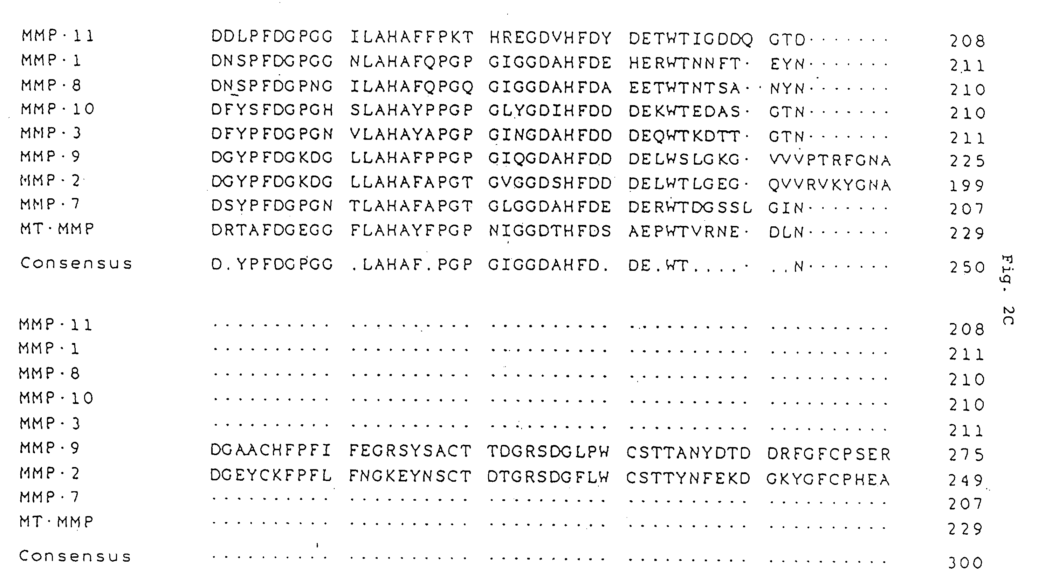

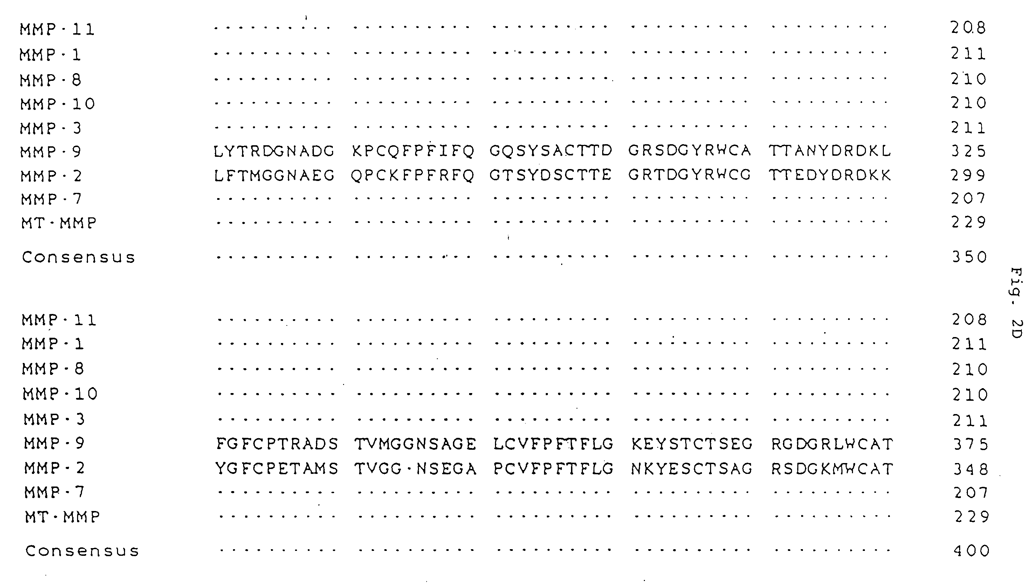

Hydrophilic and hydrophobic values of the amino acid sequence denoted by Sequence Sheet sequence number 1, as predicted from the nucleotide sequence denoted by Sequence Sheet sequence number 2, were calculated by the Kyte-Doolittle method (J. Mol. Biol., 157:105-132, 1982), and the hydrophilic and hydrophobic distribution plot shown in Figure 1 was determined. A hydrophobic domain comprising a sequence of 20 or more linked hydrophobic amino acids peculiar to a membrane binding protein was present from position 533 to position 562 of the C-terminal region of Sequence Sheet sequence number 1, and the sequence thereof is shown in Sequence Sheet sequence number 7. Such a sequence of linked hydrophobic amino acids does not exist in previously known MMPs.

When the homology of the amino acid sequence in Sequence Sheet sequence number 1 was compared to reported MMPs amino acid sequences, the amino acid sequence in Sequence Sheet sequence number 1 showed homology with the MMP family. Specifically, processing site between precursor and active enzyme and active site conserved to an extremely high degree among MMP family were each highly conserved in MT-MMP as well (Sequence Sheet sequence number 1, amino acids numbers 88-97 and 112-222).

### Working Example 2 Gene Expression

### (a) Expression in Tissues

Using ³²P-labeled PCR product A noted in Working Example 1, section (c) as a probe, hybridization was performed with poly(A)⁺ RNA blotted membrane, human multiple tissue Northern Blots (Clontech), which contain poly(A)⁺ RNA from human heart, brain, placenta, lung, liver, skeletal muscle, kidney and pancreas. Human multiple tissue Northern Blot filters wetted with 3x SSC (0.45M NaCl, 0.045M trisodium citrate·2H₂O, pH 7.0) were prehybridized for 2 to 3 hours in a prehybridization solution (0.75M NaCl, 2.5mM EDTA, 0.5x Denhardt's solution, 50% formamide and 20mM Tris-HCl buffer, pH 7.5 containing 1% SDS) with gentle agitation. Next, a heat-denatured probe was added to the hybridization solution (10% sodium dextran and 50µg/ml denatured salmon sperm DNA-containing prehybridization solution), the prehybridization solution was replaced, and hybridization was performed overnight at 43°C. After hybridization was complete, the filters were washed with 2x SSC containing 0.1% SDS. Next, the filters were placed for 15 minutes at 68°C in 1x SSC containing 0.1% SDS. The filters were then dried, layered with X-ray film (Kodak XR), and 1 week autoradiography was then carried out at -70°C. The size of the MT-MMP gene transcripts was 4.8 kb in each tissue. When the developed X-ray films were traced by a densitometer and signal intensity was measured, among the investigated tissues, MT-MMP genes were found to be highly expressed in the lung, placenta and kidney; see Fig. 3.

### (b) Expression in Tumour Tissues

Normal and tumour tissues were taken from samples of two squamous cell carcinomas human lung, respectively, and total RNA was extracted by a guanidine-cesium chloride method. 10µg of each said RNA was applied to 1% agarose electrophoresis and then transferred onto a nylon membrane. Hybridization was then carried out with the ³²P-labeled probe noted in Working Example 1, section (c). Hybridization and autoradiography tracing were performed as described in the foregoing section (a). In each human lung squamous cell carcinoma, significantly higher expression were seen in tumour tissue (see Figure 4 T) than in normal tissue (see Figure 4 N).

### Working Example 3 Preparation of Monoclonal Antibodies

### (a) Preparation of Polypeptides as Antigen

From the MT-MMP amino acid sequence denoted by Sequence Sheet sequence number 1, sequences denoted by Sequence Sheet sequence numbers 8, 9 and 10 (sequence of Sequence Sheet sequence number 1 amino acid numbers 160-173, 320-333, and 498-512, respectively; hereinafter termed polypeptide A, polypeptide B and polypeptide C, respectively) were selected as specific sequences having low homology to other members of MMP family. These polypeptides were synthesized by Fmoc-BOP method using a peptide synthesizer (MilliGen/Biosearch, Peptide Synthesizer 9600), and cysteine was introduced at the N-terminus. Each synthesized peptide was purified by high speed liquid chromatography.

### (b) Preparation of Each Polypeptides and Keyhole Limpet Hemocyanin Complexes

2 mg of keyhole limpet hemocyanin (KLH) dissolved in 1 ml of 0.1M phosphate buffer, pH 7.5 and 1.85 mg N-(ε-maleimidocaproyloxy)succinimide dissolved in 200 µl dimethylformamide were mixed and incubated at 30°C for 30 minutes. Next, the above-noted mixture was applied to gel filtration by PD-10 (Pharmacia) equilibrated with 0.1M phosphate buffer, pH 7.0. KLH-bound maleimide was collected and concentrated to less than 1.5 ml. Each polypeptide synthesized in the foregoing section (a) was respectively dissolved in 1 ml of 0.1M phosphate buffer, pH 7.0 and mixed with KLH-bound maleimide at a molar ratio representing a factor of 50. This material was then incubated 20 hours at 4°C, and KLH-polypeptide complexes were thus prepared.

### (c) Preparation of Antibody-producing Cells

As an initial immunization, eight-week-old Balb/c female mice were given an intraperitoneal administration of 250 µg of a complex of KLH and, respectively, polypeptide A, polypeptide B or polypeptide C prepared in the above section (b), in Freund's complete adjuvant. After 18 days, the respectively immunized mice were boosted intraperitoneally with 200 µg of the respective complexes dissolved in 0.1M phosphate buffer, pH 7.5. After 32 days, a final immunization of 100 µg of each complex was administered intravenously as the booster immunization. Three days thereafter, splencytes were extirpated and splencyte suspensions were prepared.

### (d) Cell Fusion

Fusion with 8-azaguanine-resistant myeloma cell SP2 (SP2/O-Ag14) was performed according to a modifying method of Oi et al (see Selected Methods in Cellular Immunology, Mishell, B.B. and Shiigi, S. M., ed., W.H. Freeman and Company pp. 351-372, 1980). Fusion of myeloma cell SP2 with karyo-splencytes from mice immunized with the polypeptide A-KLH complex is discussed in details, hereafter.

Through the following procedures, karyo-splencytes prepared in the foregoing section (c) (cell viability 100%) were fused in a 5:1 ratio with myeloma cells (cell viability 100%). A polypeptide A-immunized splencyte suspension and myeloma cells were separately washed in RPMI 1640 medium. The material was then suspended in the same medium, and 3x10⁸ cells of karyo-splencytes and 6x10⁷ cells of myeloma cells were mixed for fusion. The cells were then precipitated by centrifugation, and all the supernatant was completely discarded by suction. 2.0 ml of PEG 4000 solution (RPMI 1640 medium containing 50% [w/v] polyethylene glycol 4000) prewarmed at 37°C was added dropwise to the precipitated cells over 1 minute, 1 minute stirring was performed, and the cells were resuspended and dispersed. Next, 2.0 ml of RPMI 1640 medium prewarmed at 37°C was added in a dropwise fashion over 1 minute. After repeating the same operation once more, 14 ml of RPMI 1640 medium was added dropwise over 2 to 3 minutes under constant stirring, and the cells were dispersed. The dispersion was centrifuged and the supernatant was completely discarded by suction. Next, 30 ml of NS-1 medium (RPMI 1640 medium containing filter-sterilized 15% [w/v] fetal calf serum [JRH Biosciences]) prewarmed at 37°C was rapidly added to the precipitated cells, and the large cell clumps were carefully dispersed by pipetting. The dispersion was then diluted by adding 30 ml of NS-1 medium, and 6.0x10⁵ cells/0.1 ml/well was added to a polystyrene 96-microwell plate. The above-noted cell-filled microwells were cultured in 7% carbonic acid gas/93% atmospheric air at 37°C and 100% humidity.

In the case of splencytes derived from mice immunized with the polypeptide B-KLH complex, 6.4x10⁸ cells of splencytes and 1.28x10⁸ cells of myeloma cells were mixed, and respectively, 4.3 ml, 38.7 ml and 129 ml of the above-used PEG 4000 solution, RPMI 1640 medium and NS-1 medium were used. In the case of splencytes derived from mice immunized with the polypeptide C-KLH complex, 6.8x10⁸ cells of splencytes and 1.36x10⁸ cells of myeloma cells were mixed, and 4.5 ml, 40.5 ml and 135 ml of respectively PEG 4000 solution, RPMI 1640 medium and NS-1 medium were used.

### (e) Selective Amplification of Hybridomas by Selective Culture Medium

On the day following the start of culturing mentioned in the above section (d) (Day 1), 2 drops (approx. 0.1 ml) HAT culture medium (100 µM hypoxanthine, 0.4 µM aminopterin and 16 µM thymidine added to NS-1 culture medium) were added to the cells with a Pasteur pipette. On Days 2, 3, 5 and 8, half of each culture medium (approx. 0.1 ml) was replaced with fresh HAT medium, and on Day 11, half of each culture medium was replaced with fresh HT culture medium (HAT culture medium not containing aminopterin). On Day 14, for all the wells in which hybridoma growth was observed to the naked eye, positive wells were investigated by enzyme-linked immunoadsorbent assay (ELISA). Specifically, the polystyrene 96-well plate was respectively coated with polypeptides A, B and C serving as antigens, washed using PBS for washing (containing 0.05% Tween 20), and unadsorbed peptides were thus removed. In addition, the uncoated portion of each well was blocked with 1% BSA. 0.1 ml of supernatant from wells in which hybridoma growth was confirmed was added to each polypeptide-coated well, and the plate was stood at room temperature for approximately 1 hour.

Horseradish peroxidase-labeled goat anti-mouse immunoglobulin was added as a secondary antibody, and the plate was again stood at room temperature for approximately another 1 hour. A substrate of hydrogen peroxide and o-phenylenediamine was added, and the degree of color development was measured as absorbance at 492 nm using a microplate light absorbency measuring device (MRP-A4, Tosoh).

### (f) Hybridoma Cloning

Hybridomas in wells positive with respect to individual antigen peptides, as obtained in the foregoing section (e), were monocloned according to the limiting dilution method. Specifically, hybridomas were diluted to 5, 1 and 0.5 per well and were respectively added to 36, 36 and 24 wells of a 96 microwells. On Day 5 and Day 12, approximately 0.1 ml NS-1 medium was added to each well. Approximately 2 weeks after cloning began, the ELISA noted in section (e) was performed for groups in which sufficient hybridoma growth was visually confirmed and 50% or more wells were negative for colony formation. If all tested wells were not positive, 4 to 6 antibody-positive wells in which the number of colonies was 1 were selected, and recloning was performed. Finally, as shown in Table 1 and Table 2, 12, 20 and 9 hybridomas were obtained which produced monoclonal antibodies against polypeptide A, polypeptide B or polypeptide C, respectively.

### (g) Hybridoma Culturing and Monoclonal Antibody Purification

Each obtained hybridoma was cultured in NS-1 medium and a 10 to 100 µg/ml concentration of monoclonal antibody was successfully obtained from the supernatant thereof. In addition, BALB/c mice given an one week prior intraperitoneal administration of pristane were given a similar intraperitoneal administration of 1x10⁷ cells of obtained hybridomas, and after 1 to 2 weeks, abdominal fluid containing 4 to 7 mg/ml of monoclonal antibody was successfully obtained. The abdominal fluid obtained was salted out by 40% saturated ammonium sulfate, and IgG class antibodies were adsorbed to Protein A Affigel (Bio-Rad) and purified by elution with a 0.1M citric acid buffer, pH 5.

### (h) Determination of Monoclonal Antibody Class and Subclass

In accordance with the above-discussed ELISA, the supernatant of monoclones obtained in section (f) were added to microtitration plates respectively coated with polypeptide A, polypeptide B or polypeptide C. After washing with PBS, isotype-specific rabbit anti-mouse IgG antibodies (Zymed Lab.) were added. After washing with PBS, horseradish peroxidase-labeled goat anti-rabbit IgG (H+L) was added, and class and subclass were determined using hydrogen peroxide and 2.2'-azino-di(3-ethylbenzthiazolinic acid) as a substrate.

### (i) Specificity of Anti-MT-MMP Monoclonal Antibodies

The cross-reactivity of five varieties of anti-MT-MMP monoclonal antibodies (monoclone numbers 113-5B7, 113-15E7, 114-1F1, 114-2F2 and 118-3B1) exhibiting a positive reaction 'against a human MT-MMP peptide was determined by the ELISA noted in the foregoing section (e), using as respective antigens: proMMP-1 (Clin, Chim. Acta, 219:1-14, 1993), proMMP-2 (Clin. Chim. Acta, 221:91-103, 1993) and proMMP-3 (Clin. Chim. Acta, 211:59-72, 1992) respectively purified from the supernatant of normal human skin fibroblast (NBlRGB) culture; proMMP-7 purified from the supernatant of human rectal carcinoma cell (Car-1) culture (Cancer Res., 50:7758-7764, 1990), proMMP-8 purified from human neutrophils (Biol. Chem. Hoppe-Seyler, 371 supp:295-304, 1990) and proMMP-9 purified from the supernatant of human fibrosarcoma cells (HT1080) culture (J. Biol. Chem., 267: 21712-21719, 1992).

Specifically, using a polystyrene 96-well plate, each well was coated by adding 50. ng/well of purified MMP-1, MMP-2, MMP-3, MMP-7, MMP-8 and MMP-9, respectively. Washing was performed with PBS for washing and non-adsorbed antigen was removed, and the uncoated portion of each well was blocked with PBS containing 3% skim milk. 1 µg/well of each anti-MT-MMP monoclonal antibody was respectively added to each well and stood at room temperature for approximately 1 hour. After washing plate, peroxidase-labeled goat anti-mouse immunoglobulin was added as a secondary antibody, and the plate was again stood at room temperature for approximately 1 hour. A substrate of hydrogen peroxide and o-phenylene diamine was added, and the degree of color development was measured absorbance at 492 nm using a microplate light absorbency measuring device (MRP-A4, Tosoh).

In results, as shown in Table 3, each anti-MT-MMP monoclonal antibody showed no reactivity against purified MMPs other than the MT-MMP supplied for testing.

**TABLE 1**

| Polypeptide | Monoclone No. | Subclass/Chain |
|---|---|---|
| A | 114-1F2 | γ1/κ |
| | 114-2F2 | γ1/κ |
| | 114-3H7 | γ1/κ |
| | 114-5E4 | γ1/κ |
| | 114-6G6 | γ1/κ |
| | 114-8D10 | γ1/κ |
| | 114-9H3 | µ/κ |
| | 114-15E8 | γ1/κ |
| | 114-16C11 | γ1/κ |
| | 114-18E4 | γ1/κ |
| | 114-19F11 | γ1/κ |
| | 114-20H5 | µ/κ |
| B | 113-1E3 | γ3/κ |
| | 113-2E9 | γ3/κ |
| | 113-3F6 | γ2b/κ |
| | 113-4H7 | γ3/κ |
| | 113-5B7 | γ3/κ |
| | 113-7C6 | γ2b/κ |
| | 113-9G9 | γ3/κ |
| | 113-10F2 | γ3/κ |
| | 113-13G11 | γ3/κ |
| | 113-15E7 | γ3/κ |
| | 113-16H8 | γ3/κ |
| | 113-17G12 | µ/κ |
| | 113-19A10 | µ/κ |
| | 113-20G11 | γ3/κ |
| | 113-21H3 | γ1/κ |
| | 113-26D3 | µ/κ |
| | 113-44C1 | γ1/κ |
| | 113-46B7 | γ1/κ |
| | 113-53G5 | µ/κ |
| | 113-63E8 | γ1/κ |

**TABLE 2**

| Polypeptide | Monoclone No. | Subclass/Chain |
|---|---|---|
| C | 118-3B1 | γ2b/κ |
| | 118-6F3 | γ2b/κ |
| | 118-8D11 | γ1/κ |
| | 118-9B11 | γ1/κ |
| | 118-13D11 | α/κ |
| | 118-18C12 | γ1/κ |
| | 118-20A3 | γ2b/κ |
| | 118-25C3 | γ1/κ |
| | 118-26F5 | γ3/κ |

**TABLE 3**

| Monoclone No. | Cross reactivity | | | | | |
|---|---|---|---|---|---|---|
| | MMP-1 | MMP-2 | MMP-3 | MMP-7 | MMP-8 | MMP-9 |
| 113-5B7 | - | - | - | - | - | - |
| 113-15E7 | - | - | - | - | - | - |
| 114-1F2 | - | - | - | - | - | - |
| 114-2F2 | - | - | - | - | - | - |
| 118-3B1 | - | - | - | - | - | - |
| - :No reaction | | | | | | |

### Working Example 4 Expression and Identification of Gene Product

By means of EcoR I cleavage, an insertion fragment was excised from the recombinant pBluescript containing a cloned MT-MMP gene, constructed in section (f) of Working Example 1. Cloning was then carried out at an EcoR I site of the eukaryotic expression vector pSG5 (Stratagene). Then, human fibrosarcoma cells (HT1080) were transfected with said recombinant pSG5 by a calcium phosphate method. Specifically, 20 µg of recombinant pSG5 and 62 µl of 2M CaCl₂ was added to distilled water, and 2x HBSP solution (50mM HEPES buffer, pH 7.1 containing 1.5mM Na₂HPO₄, 10mM KCl, 280mM NaCl and 12mM glucose) was added to the bottom of the tube to form a total volume of 1 ml. This material was mixed, stood at room temperature for approximately 30 minutes, and thorough precipitate formation was carried out. The precipitate was dispersed by pipetting, added dropwise to HT1080 cells and incubated for approximately 4 hours in a CO₂ incubator. Next, the culture medium was removed, a 15% glycerol solution was added and treated for 1 to 3 hours, the glycerol was discarded by suction, washed with PBS and fresh culture medium containing ³⁵S-methionine was added. Culturing was continued, and cellular proteins were labeled by ³⁵S. Incidentally, expression of MT-MMP genes in HT1080 cells cannot be detected by Northern Blot analysis.

The cells were incubated for 1 hour at 4°C in a lysing buffer solution (0.01M Tris-HCl buffer, pH 8 containing 1% Triton X-100, 1% bovine hemoglobin, 1mM iodoacetamide, 0.2U trypsin inhibitor, 1mM PMSF and 0.14M NaCl). The cell lysate was centrifuged and the supernatant was recovered. Sepharose-4B (Pharmacia) coupled with a monoclonal antibody obtained in Working Example 3 was added to the supernatant, the material was incubated at 4°C for 2 hours with agitation, and immunoprecipitation was carried out. Monoclonal antibodies against polypeptide A used in immunoprecipitation were two of the 12 obtained in Working Example 3 which had low non-specific reactivity (monoclone numbers 114-1F2 and 114-2F2 [Assignment No. FERM BP-4743]). Next, Sepharose 4B coupled with monoclonal antibodies subjected to immunoprecipitation were precipitated by centrifugation, washed three times with a washing solution (0.01M Tris-HCl buffer, pH 8 containing 1% Triton X-100, 1% bovine hemoglobin and 0.14M NaCl), and lastly, washed with 0.05M Tris-HCl buffer, pH 6.8. A sample buffer for SDS polyacrylamide electrophoresis was added to washed Sepharose-4B coupled with a monoclonal antibody, boiled 5 minutes at 100°C, and SDS polyacrylamide electrophoresis was carried out. The electrophoresed gel was layered with X-ray film (Kodak XR), 1 week autoradiography was then carried out at -70°C, and the developed X-ray films were traced by a densitometer to measure signal intensity. With each of the anti-MT-MMP monoclonal antibodies used (monoclone numbers 114-1F2 and 114-2F2), the immunoprecipitate contained a 63 kDa protein. In cells transfected with a pSG5 vector alone not containing an MT-MMP gene as a control, anti-MT-MMP monoclonal antibodies (monoclone numbers 114-1F2 and 114-2F2) did not precipitate a 63 kDa protein. The 63 kDa molecular weight of the protein detected by immunoprecipitation nearly matched a molecular weight of 65.78 kDa calculated from the amino acid sequence denoted by Sequence Sheet sequence number 1. In addition, a variant MT-MMP expression plasmid was constructed in which amino acids from position 13 to position 101 were deleted from the amino acid sequence denoted by Sequence Sheet sequence number 1. HT1080 cells were transfected with said variant as stated above, and immunoprecipitation was carried out. With HT1080 cells to which the variant MT-MMP gene was introduced, a 63 kDa protein was not detected, and a 55 kDa protein was detected. This molecular weight matched a molecular weight predicted from the introduced deletion.

### EXPERIMENTAL EXAMPLE

### (a) Activation of proMMP-2 by MT-MMP Expression

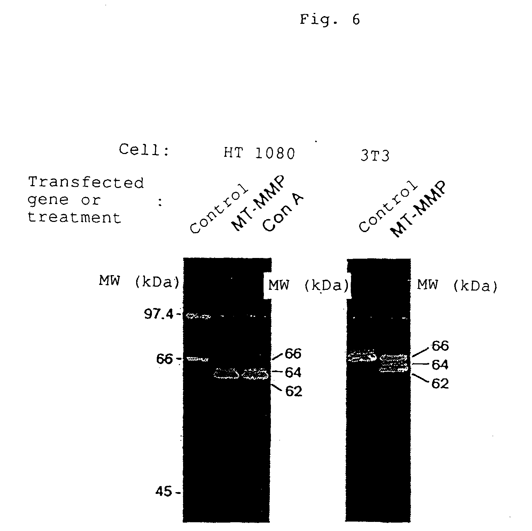

Recombinant pSG5 carrying a cloned MT-MMP gene, constructed in Working Example 4, and a pSG5 vector alone, serving as a control, similarly transfected into HT1080 cells by the calcium phosphate method mentioned in Working Example 4, or into mouse embryonic fibroblasts NIH3T3. However, a regular fresh culture medium was used in lieu of the fresh culture medium containing ³⁵S-methionine. Both the HT1080 cells and the NIH3T3 cells secreted proMMP-2 and proMMP-9 (corresponding respectively to the 66 kDa and 97.4 kDa bands in Figure 6), and in cells transfected with an MT-MMP gene, MT-MMP expression was confirmed by immunoprecipitation experiments (See Working Example 4).

The transfectants obtained were cultured for 24 hours in a serum free medium and the recovered culture supernatant was supplied for zymography. The culture supernatant was mixed with an SDS polyacrylamide electrophoresis buffer (non-reducing condition) and left at 4°C overnight. Electrophoresis was then performed at 4°C, with a 20 mA current, using a 10% polyacrylamide gel containing 1 mg/ml casein. After electrophoresis, the gel was washed with a gelatinase-buffer (Tris-HCl buffer, pH 7.6 containing 5mM CaCl₂ and 1 µM ZnSO₄) containing 2.5% Triton X-100 with gentle agitation for 15 minutes, and this operation was repeated twice. Next, the gel was immersed in a gelatinase-buffer containing 1% Triton X-100 and stood at 37°C overnight. The buffer was discarded and the gel was stained for 1 hour with 0.02% Coomassie Brilliant Blue-R (dissolved in 50% methanol/10% acetic acid) and destained by immersion in a destaining solution (5% methanol, 7.5% acetic acid).

As shown in Figure 6, MT-MMP gene-transfected HT1080 cells produced new 64 kDa and 62 kDa bands, confirming proMMP-2 activation. This active-form MMP-2 exhibited the same molecular weight as an active-form MMP-2 molecule induced by treatment of cells with 100 µg/ml of concanavalin A and reacted specifically against anti-MMP-2 monoclonal antibodies. This activation was not observed in a control transfected with a vector alone. Likewise, proMMP-9 showed no change in molecular weight and no activation similar to that observed in control cells. Such activation of proMMP-2 depending on MT-MMP expression was also observed in MT-MMP gene-transfected NIH3T3 cells.

### (b) Activation of ProMMP-2 by MT-MMP Expression Cell Membrane Fraction

In a manner similar to that noted in the above section (a), African green monkey kidney-derived COS-1 cells were transfected with recombinant pSG5 containing cloned MT-MMP gene, or with control pSG5 vector alone by a calcium phosphate method. A cell membrane fraction was then prepared from the obtained transfectant according to the method of Strongin et al. (J. Biol. Chem., 268:14033-14039, 1993).

The transfectant was washed with PBS, and cells were harvested by centrifugation and suspended in a 25mM Tris-HCl buffer, pH 7.4 containing 8.5% sucrose, 50mM NaCl, 10mM N-ethylmaleimide, 10 µg/ml aprotinin, 1 µg/ml pepstatin A, 1 µg/ml leupeptin and 1mM phenylmethylsulfonyl fluoride. The cell suspension was homogenized in a Dounce homogenizer, and the homogenate was centrifuged (3000x g, 10 min., 4°C). The resulting supernatant was ultracentrifuged (100,000x g, 2 hours) and the precipitate was suspended in a 25mM Tris-HCl buffer, pH 7.4 containing 50mM NaCl, 10mM N-ethylmaleimide, 10 µg/ml aprotinin, 1 µg/ml pepstatin A, 1 µg/ml leupeptin and 1mM phenylmethylsulfonyl fluoride. This suspension was fractionated by discontinuous sucrose density gradient centrifugation (20, 30, 50, 60% sucrose solutions; 100,000x g; 2 hours; 4°C), and bands of cell membrane fractions appeared were recovered. These fractions were precipitated again by ultracentrifugation (100,000x g, 2 hours), suspended in 25mM HEPES/KOH buffer, pH 7.5 containing 0.1mM CaCl₂ and 0.25% Triton X-100, and adjusted to a final protein concentration of 1-2 mg/ml. This suspension was ultracentrifuged (100,000x g, 1.5 hours, 4°C) to remove insoluble residue, and the supernatant obtained was taken as a cell membrane fraction.

Cell membrane fractions (protein content 20 µg) respectively prepared from untreated COS-1 cells or from COS-1 cells transfected with pSG5 vector alone or pSG5 vector with an MT-MMP gene were incubated with HT1080 cell culture supernatant at 37°C for 2 hours. Using these samples, the zymography noted in the above section (a) was performed.

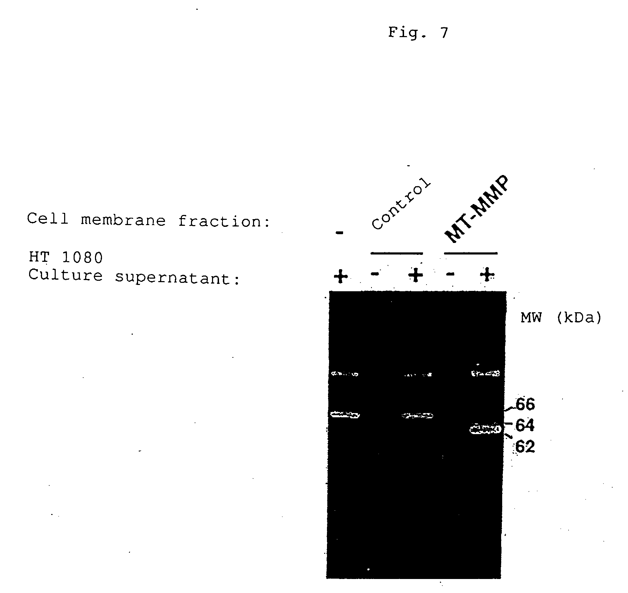

In the results, new 64 kDa and 62 kDa bands appeared and the activation of proMMP-2 present in HT1080 cell culture supernatant was observed only when cell membrane fractions derived from MT-MMP gene-transfected COS-1 cells were used (see Figure 7), and the activation of proMMP-2 was inhibited by the addition of recombinant (r) human TIMP-2. These results exhibited the activation of proMMP-2 by MT-MMP expressed on a cell membrane.

### (c) Stimulation of cellular invasion in vitro due to MT-MMP expression

Invasion of cells was assayed by modified Boyden Chamber method (Cancer Res., 47:3239-3245, 1987), and operations were carried out in accordance with the manufucture's instructions for a Biocoat Matrigel Invasion Chamber (Becton Dickinson).

In a manner similar to that noted in the foregoing section (a), HT1080 cells or NIH3T3 cells were transfected with recombinant pSG5 carrying a cloned MT-MMP gene, or a control pSG5 vector alone, by a calcium phosphate method, and each of these host cells secreted proMMP-2. The resulting transfectants were then suspended in DMEM medium containing 0.1% BSA, and 2x10⁵ cells were seeded onto an uncoated filter (pore size 8 µm) or a preswelled Matrigel Coat filter in a Biocoat Matrigel Invasion Chambers. After 24 hours incubation in a CO₂ incubator at 37°C, the filters were fixed by 10 seconds immersion in methanol. The filters were then stained by hematoxylin for 3 minutes, washed, and stained by eosin for 10 seconds, and the number of cells invaded the bottom surface of the filters were counted under a light microscope (at a magnification of x 400).

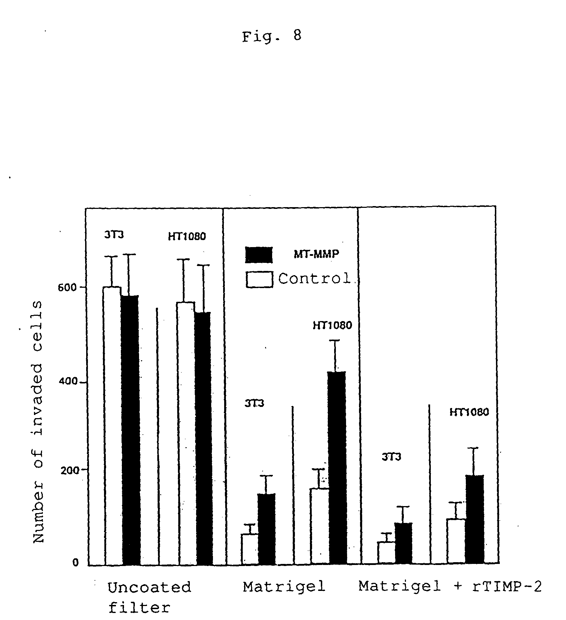

In the MT-MMP gene-transfected HT1080 cells and NIH3T3 cells, more than twice as many invading cells were seen compared to cells transfected with the control vector alone (See Figure 8 Matrigel). Specifically, MT-MMP expression was seen to stimulate cellular invasion. Furthermore, the addition of 10 µg/ml of r-human TIMP-2 to this assay system clearly suppressed cellular invasion (see Figure 8 Matrigel+r-human TIMP-2).

### BRIEF DESCRIPTION OF THE FIGURES

Figure 1 shows hydrophilic and hydrophobic distribution diagrams for the amino acid sequence of MT-MMP, according to the Kyte-Doolittle method.

Figures 2A, 2B, 2C, 2D, 2E, 2F, 2G and 2H are figures comparing sequential homology between the amino acid sequences of MT-MMP and those of the known MMP family (MMP-1, MMP-2, MMP-3, MMP-7, MMP-8, MMP-9, MMP-10 and MMP-11). Letters in each figure indicate respective amino acids; A corresponding to Ala, C to Cys, D to Asp, E to Glu, F to Phe, G to Gly, H to His, I to Ile, K to Lys, L to Leu, M to Met, N to Asn, P to Pro, Q to Gln, R to Arg, S to Ser, T to Thr, V to Val, W to Trp and Y to Tyr. Figures 2A through 2H are an integral unit and comprise a single figure.

Figure 3 shows a relative expression of MT-MMP mRNA in various human tissues, according to Northern blot analysis.

Figure 4 shows a relative expression of MT-MMP mRNA in a normal tissue and a tumour tissue of two samples of human lung squamous cell carcinoma, according to Northern blot analysis.

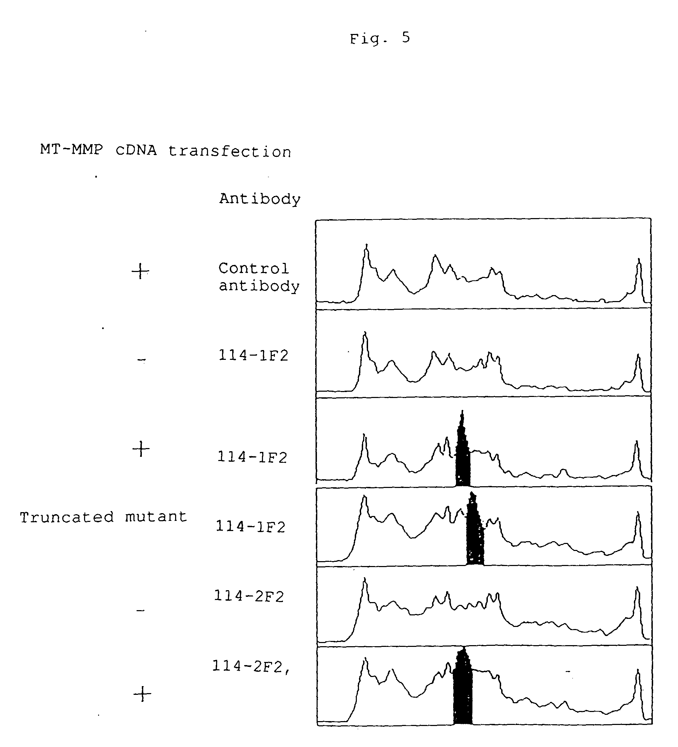

Figure 5 shows results for detection, by immunoprecipitation, of MT-MMP proteins expressed in HT1080 cells transfected with MT-MMP cDNA. The figure shows a scan by a densitometer, and the darkened areas indicate the location of MT-MMP immunoprecipitated by anti-MT-MMP monoclonal antibody.

Figure 6 shows an activation of proMMP-2 by expression of MT-MMP, according to zymography of culture supernatant from HT1080 and NIH3T3 cells transfected with MT-MMP cDNA.

Figure 7 shows an activation of proMMP-2 by a cell membrane fraction of COS-1 cells transfected with MT-MMP cDNA, according to zymography.

Figure 8 shows a stimulation of the cellular invasion by expression of MT-MMP, according to a partially modified Boyden chamber method.

### SEQUENCE LISTING

### GENERAL INFORMATION

APPLICANT
NAME: Fuji Yakuhin Kogyo Kabushiki Kaisha
STREET:530 Chokeiji, Takaoka-shi
CITY: Toyama-ken
COUNTRY: Japan
POSTAL CODE: 933

### TITEL OF INVENTION:

Novel metalloproteinase and DNA coding for the same
NUMBER OF SEQUENCES: 10
COMPUTER READABLE FORM
MEDIUM TYPE: Diskette, 3,5inch, 1,44MB
COMPUTER:IBM PC compatible
OPERATING SYSTEM: PC-DOS/MS-DOS
SOFTWARE: Word 5.5/DOS

### CURRENT APPLICATION DATA

APPLICATION NUMBER: EP 95 90 2274.0-2105

### PRIOR APPLICATION DATA

APPLICATION NUMBER: PCT/JP94/02009
INFORMATION FOR SEQ ID NO: 1
   LENGTH: 582
   TYPE: Amino acid
   TOPOLOGY: Linear
   MOLECULE TYPE: Protein
   SEQUENCE DESCRIPTION: SEQ ID NO:1 
Met Ser Pro Ala Pro Arg Pro Ser Arg Cys Leu Leu Leu Pro Leu
1               5                   10                  15

Leu Thr Leu Gly Thr Ala Leu Ala Ser Leu Gly Ser Ala Gln Ser
                20  -               25                  30

Ser Ser Phe Ser Pro Glu Ala Trp Leu Gln Gln Tyr Gly Tyr Leu
                35                  40                  45

Pro Pro Gly Asp Leu Arg Thr His Thr Gln Arg Ser Pro Gln Ser
                50                  55                  60

Leu Ser Ala Ala Ile Ala Ala Met Gln Lys Phe Tyr Gly Leu Gln
                65                  70                  75

Val Thr Gly Lys Ala Asp Ala Asp Thr Met Lys Ala Met Arg Arg
                80                  85                  90
 
Pro Arg Cys Gly Val Pro Asp Lys Phe Gly Ala Glu Ile Lys Ala
95 100 105

Asn Val Arg Arg Lys Arg Tyr Ala Ile Gln Gly Leu Lys Trp Gln
110 115 120

His Asn Glu Ile Thr Phe Cys Ile Gln Asn Tyr Thr Pro Lys Val
125 130 135

Gly Glu Tyr Ala Thr Tyr Glu Ala Ile Arg Lys Ala Phe Arg Val
140 145 150

Trp Glu Ser Ala Thr Pro Leu Arg Phe Arg Glu Val Pro Tyr Ala
155 160 165

Tyr Ile Arg Glu Gly His Glu Lys Gln Ala Asp Ile Met Ile Phe
170 175 180

Phe Ala Glu Gly Phe His Gly Asp Arg Thr Ala Phe Asp Gly Glu
185 190 195

Gly Gly Phe Leu Ala His Ala Tyr Phe Pro Gly Pro Asn Ile Gly
200 205 210

Gly Asp Thr His Phe Asp Ser Ala Glu Pro Trp Thr Val Arg Asn
215 220 225

Glu Asp Leu Asn Gly Asn Asp Ile Phe Leu Val Ala Val His Glu
230 235 240

Leu Gly His Ala Leu Gly Leu Glu His Ser Ser Asp Pro Ser Ala
245 250 255

Ile Met Ala Pro Phe Tyr Gln Trp Met Asp Thr Glu Lys Phe Val
260 265 270

Leu Pro His Tyr Asp Pro Arg Gly Ile Gln Gln Leu Tyr Gly Gly
275 280 285

Lys Gln Gly Ser Pro Pro Arg Cys Pro Leu Asn Pro Gly Leu Pro
290 295 300

Pro Gly Leu Leu Phe Leu Ile Asn Pro Lys Asn Pro Thr Tyr Gly
305 310 315

Pro Asn Ile Cys Asp Gly Asn Phe Asp Thr Val Ala Met Leu Arg
320 325 330

Gly Glu Met Phe Asp Phe Lys Lys Arg Trp Phe Trp Arg Val Arg
335 340 345

Asn Asn Gln Val Met Asp Gly Tyr Pro Met Pro Ile Gly Gln Phe
350 355 360

Trp Arg Gly Leu Pro Ala Ser Ile Asn Thr Ala Tyr Glu Arg Lys
 
365 370 375

Asp Gly Lys Phe Val Phe Phe Lys Gly Asp Lys His Trp Val Phe
380 385 390

Asp Glu Ala Ser Leu Glu Pro Gly Tyr Pro Lys His Ile Lys Glu
395 400 405

Leu Gly Arg Gly Leu Pro Thr Asp Lys Ile Asp Ala Ala Leu Phe
410 415 420

Trp Met Pro Asn Gly Lys Thr Tyr Phe Phe Arg Gly Asn Lys Tyr
425 430 435

Tyr Arg Phe Asn Glu Glu Leu Arg Ala Val Asp Ser Glu Tyr Pro
440 445 450

Lys Asn Ile Lys Val Trp Glu Gly Ile Pro Glu Ser Pro Arg Gly
455 460 465

Ser Phe Met Gly Ser Asp Glu Val Phe Thr Tyr Phe Tyr Lys Gly
470 475 480

Asn Lys Tyr Trp Lys Phe Asn Asn Gln Lys Leu Lys Val Glu Pro
485 490 495

Gly Tyr Pro Lys Ser Ala Leu Arg Asp Trp Met Gly Cys Pro Ser
500 505 510

Gly Gly Arg Pro Asp Glu Gly Thr Glu Glu Glu Thr Glu Val Ile
515 520 525

Ile Ile Glu Val Asp Glu Glu Gly Gly Gly Ala Val Ser Ala Ala
530 535 540

Ala Val Val Leu Pro Val Leu Leu Leu Leu Leu Val Leu Ala Val
545 550 555

Gly Leu Ala Val Phe Phe Phe Arg Arg His Gly Thr Pro Arg Arg
560 565 570

Leu Leu Tyr Cys Gln Arg Ser Leu Leu Asp Lys Val
575 580
INFORMATION FOR SEQ ID NO: 2
   LENGTH: 3403
   TYPE: Nucleic acid
   STRANDEDNESS: Double strand
   TOPOLOGY: Linear
   MOLECULE TYPE: cDNA to mRNA
   ORIGINAL SOURCE:
   ORGANISM: Human
   TISSUE TYPE: Placenta SEQUENCE DESCRIPTION: SEQ ID NO:2 
AGTTCAGTGC CTACCGAAGA CAAAGGCGCC CCGAGGGAGT GGCGGTGCGA CCCCAGGGCG 60

TGGGCCCGGC CGCGGAGCCA CACTGCCCGG CTGACCCGGT GGTCTCGGAC C ATG TCT CCC 120
Met Ser Pro
1

GCC CCA AGA CCC TCC CGT TGT CTC CTG CTC CCC CTG CTC ACG CTC GGC 168
Ala Pro Arg Pro Ser Arg Cys Leu Leu Leu Pro Leu Leu Thr Leu Gly
    5                   10                  15

ACC GCG CTC GCC TCC CTC GGC TCG GCC CAA AGC AGC AGC TTC AGC CCC 216
Thr Ala Leu Ala Ser Leu Gly Ser Ala Gln Ser Ser Ser Phe Ser Pro
20                  25                  30                  35

GAA GCC TGG CTA CAG CAA TAT GGC TAC CTG CCT CCC GGG GAC CTA CGT 264
Glu Ala Trp Leu Gln Gln Tyr Gly Tyr Leu Pro Pro Gly Asp Leu Arg
                40                  45                  50

ACC CAC ACA CAG CGC TCA CCC CAG TCA CTC TCA GCG GCC ATC GCT GCC 312
Thr His Thr Gln Arg Ser Pro Gln Ser Leu Ser Ala Ala Ile Ala Ala
            55                  60                  65

ATG CAG AAG TTT TAC GGC TTG CAA GTA ACA GGC AAA GCT GAT GCA GAC 360
Met Gln Lys Phe Tyr Gly Leu Gln Val Thr Gly Lys Ala Asp Ala Asp
        70                  75                  80

ACC ATG AAG GCC ATG AGG CGC CCC CGA TGT GGT GTT CCA GAC AAG TTT 408
Thr Met Lys Ala Met Arg Arg Pro Arg Cys Gly Val Pro Asp Lys Phe
    85                  90                  95

GGG GCT GAG ATC AAG GCC AAT GTT CGA AGG AAG CGC TAC GCC ATC CAG 456
Gly Ala Glu Ile Lys Ala Asn Val Arg Arg Lys Arg Tyr Ala Ile Gln
100                 105                 110                 115

GGT CTC AAA TGG CAA CAT AAT GAA ATT ACT TTC TGC ATC CAG AAT TAC 504
Gly Leu Lys Trp Gln His Asn Glu Ile Thr Phe Cys Ile Gln Asn Tyr
                120                 125                 130

ACC CCC AAG GTG GGC GAG TAT GCC ACA TAC GAG GCC ATT CGC AAG GCG 552
Thr Pro Lys Val Gly Glu Tyr Ala Thr Tyr Glu Ala Ile Arg Lys Ala
            135                 140                 145

TTC CGC GTG TGG GAG AGT GCC ACA CCA CTG CGC TTC CGC GAG GTG CCC 600
Phe Arg Val Trp Glu Ser Ala Thr Pro Leu Arg Phe Arg Glu Val Pro
        150                 155                 160

TAT GCC TAC ATC CGT GAG GGC CAT GAG AAG CAG GCC GAC ATC ATG ATC 648
Tyr Ala Tyr Ile Arg Glu Gly His Glu Lys Gln Ala Asp Ile Met Ile
    165                 170                 175
 
TTC TTT GCC GAG GGC TTC CAT GGC GAC AGG ACG GCC TTC GAT GGT GAG 696
Phe Phe Ala Glu Gly Phe His Gly Asp Arg Thr Ala Phe Asp Gly Glu
180                 185                 190                 195

GGC GGC TTC CTG GCC CAT GCC TAC TTC CCA GGG CCC AAC ATT GGA GGA 744
Gly Gly Phe Leu Ala His Ala Tyr Phe Pro Gly Pro Asn Ile Gly Gly
                200                 205                 210

GAC ACC CAC TTT GAC TCT GCC GAG CCT TGG ACT GTC AGG AAT GAG GAT 792
Asp Thr His Phe Asp Ser Ala Glu Pro Trp Thr Val Arg Asn Glu Asp
            215                 220                 225

CTG AAT GGA AAT GAC ATC TTC CTG GTG GCT GTG CAC GAG CTG GGC CAT 840
Leu Asn Gly Asn Asp Ile Phe Leu Val Ala Val His Glu Leu Gly His
        230                 235                 240

GCC CTG GGG CTC GAG CAT TCC AGT GAC CCC TCG GCC ATC ATG GCA CCC 888
Ala Leu Gly Leu Glu His Ser Ser Asp Pro Ser Ala Ile Met Ala Pro
    245                 250                 255

TTT TAC CAG TGG ATG GAC ACG GAA AAA TTT GTG CTT CCC CAT TAT GAC 936
Phe Tyr Gln Trp Met Asp Thr Glu Lys Phe Val Leu Pro His Tyr Asp
260                 265                 270                 275

CCC CGG GGC ATC CAG CAA CTT TAT GGG GGT AAG CAA GGT TCC CCA CCA 984
Pro Arg Gly Ile Gln Gln Leu Tyr Gly Gly Lys Gln Gly Ser Pro Pro
                280                 285                 290

AGA TGC CCC CTC AAC CCA GGA CTA CCT CCC GGC CTT CTG TTC CTG ATA 1032
Arg Cys Pro Leu Asn Pro Gly Leu Pro Pro Gly Leu Leu Phe Leu Ile
            295                 300                 305

AAC CCC AAA AAC CCC ACC TAT GGG CCC AAC ATC TGT GAC GGG AAC TTT 1080
Asn Pro Lys Asn Pro Thr Tyr Gly Pro Asn Ile Cys Asp Gly Asn Phe
        310                 315                 320

GAC ACC GTG GCC ATG CTC CGA GGG GAG ATG TTT GAC TTC AAG AAG CGC 1128
Asp Thr Val Ala Met Leu Arg Gly Glu Met Phe Asp Phe Lys Lys Arg
    325                 330                 335

TGG TTC TGG CGG GTG AGG AAT AAC CAA GTG ATG GAT GGA TAC CCA ATG 1176
Trp Phe Trp Arg Val Arg Asn Asn Gln Val Met Asp Gly Tyr Pro Met
340                 345                 350                 355

CCC ATT GGC CAG TTC TGG CGG GGC CTG CCT GCG TCC ATC AAC ACT GCC 1224
Pro Ile Gly Gln Phe Trp Arg Gly Leu Pro Ala Ser Ile Asn Thr Ala
                360                 365                 370

TAC GAG AGG AAG GAT GGC AAA TTC GTC TTC TTC AAA GGA GAC AAG CAT 1272
Tyr Glu Arg Lys Asp Gly Lys Phe Val Phe Phe Lys Gly Asp Lys His
            375                 380                 385

TGG GTG TTT GAT GAG GCG TCC CTG GAA CCT GGC TAC CCC AAG CAC ATT 1320
Trp Val Phe Asp Glu Ala Ser Leu Glu Pro Gly Tyr Pro Lys His Ile
        390                 395                 400
 
AAG GAG CTG GGC CGA GGG CTG CCT ACC GAC AAG ATT GAT GCT GCT CTC 1368
Lys Glu Leu Gly Arg Gly Leu Pro Thr Asp Lys Ile Asp Ala Ala Leu
    405                 410                 415

TTC TGG ATG CCC AAT GGA AAG ACC TAC TTC TTC CGT GGA AAC AAG TAC 1416
Phe Trp Met Pro Asn Gly Lys Thr Tyr Phe Phe Arg Gly Asn Lys Tyr
420                 425                 430                 435

TAC CGT TTC AAC GAA GAG CTC AGG GCA GTG GAT AGC GAG TAC CCC AAG 1464
Tyr Arg Phe Asn Glu Glu Leu Arg Ala Val Asp Ser Glu Tyr Pro Lys
                440                 445                 450

AAC ATC AAA GTC TGG GAA GGG ATC CCT GAG TCT CCC AGA GGG TCA TTC 1512
Asn Ile Lys Val Trp Glu Gly Ile Pro Glu Ser Pro Arg Gly Ser Phe
            455                 460                 465

ATG GGC AGC GAT GAA GTC TTC ACT TAC TTC TAC AAG GGG AAC AAA TAC 1560

        470                 475                 480

TGG AAA TTC AAC AAC CAG AAG CTG AAG GTA GAA CCG GGC TAC CCC AAG 1608

    485                 490                 495

TCA GCC CTG AGG GAC TGG ATG GGC TGC CCA TCG GGA GGC CGG CCG GAT 1656

500                 505                 510                 515

GAG GGG ACT GAG GAG GAG ACG GAG GTG ATC ATC ATT GAG GTG GAC GAG 1704

                520                 525                 530

GAG GGC GGC GGG GCG GTG AGC GCG GCT GCC GTG GTG CTG CCC GTG CTG 1752

            535                 540                 545

CTG CTG CTC CTG GTG CTG GCG GTG GGC CTT GCA GTC TTC TTC TTC AGA 1800

        550                 555                 560

CGC CAT GGG ACC CCC AGG CGA CTG CTC TAC TGC CAG CGT TCC CTG CTG 1848

    565                 570                 575

GAC AAG GTC TGACGCCCAT CCGCCGGCCC GCCCACTCCT ACCACAAGGA CTTTGCCTCT 1907

580

GAAGGCCAGT GGCAGCAGGT GGTGGTGGGT GGGCTGCTCC CATCGTCCCG AGCCCCCTCC 1967

CCGCAGCCTC CTTGCTTCTC TCTGTCCCCT GGCTGGCCTC CTTCACCCTG ACCGCCTCCC 2027

TCCCTCCTGC CCCGGCATTG CATCTTCCCT AGATAGGTCC CCTGAGGGCT GAGTGGGAGG 2087
 
GCGGCCCTTT CCAGCCTCTG CCCCTCAGGG GAACCCTGTA GCTTTGTGTC TGTCCAGCCC 2147
CATCTGAATG TGTTGGGGGC TCTGCACTTG AAGGCAGGAC CCTCAGACCT CGCTGGTAAA 2207
GGTCAAATGG GGTCATCTGC TCCTTTTCCA TCCCCTGACA TACCTTAACC TCTGAACTCT 2267
GACCTCAGGA GGCTCTGGGG AACTCCAGCC CTGAAAGCCC CAGGTGTACC CAATTGGCAG 2327
CCTCTCACTA CTCTTTCTGG CTAAAAGGAA TCTAATCTTG TTGAGGGTAG AGACCCTGAG 2387
ACAGTGTGAG GGGGTGGGGA CTGCCAAGCC ACCCTAAGAC CTTGGGAGGA AAACTCAGAG 2447
AGGGTCTTCG TTGCTCAGTC AGTCAAGTTC CTCGGAGATC TTCCTCTGCC TCACCTACCC 2507
CAGGGAACTT CCAAGGAAGG AGCCTGAGCC ACTGGGGACT AAGTGGGCAG AAGAAACCCT 2567
TGGCAGCCCT GTGCCTCTCG AATGTTAGCC TTGGATGGGG CTTTCACAGT TAGAAGAGCT 2627
GAAACCAGGG GTGCAGCTGT CAGGTAGGGT GGGGCCGGTG GGAGAGGCCC GGGTCAGAGC 2687
CCTGGGGGTG AGCCTTAAGG CCACAGAGAA AGAACCTTGC CCAAACTCAG GCAGCTGGGG 2747
CTGAGGCCCA AAGGCAGAAC AGCCAGAGGG GGCAGGAGGG GACCAAAAAG GAAAATGAGG 2807
ACGTGCAGCA GCATTGGAAG GCTGGGGCCC GGCAGCCAGG TTAAAGCTAA CAGGGGGCCA 2867
TCAGGGTGGG CTTGTGGAGC TCTCAGGAAG GGCCCTGAGG AAGGCACACT TGCTCCTGTT 2927
GGTCCCTGTC CTTGCTGCCC AGGCAGGGTG GAGGGGAAGG GTAGGGCAGC CAGAGAAAGG 2987
AGCAGAGAAG GCACACAAAC GAGGAATGAG GGGCTTCACG AGAGGCCACA GGGCCTGGCT 3047
GGCCACGCTG TCCCGGCCTG CTCACCATCT CAGTGAGGGA CAGGAGCTGG GGCTGCTTAG 3107
GCTGGGTCCA CGCTTCCCTG GTGCCAGCAC CCCTCAAGCC TGTCTCACCA GTGGCCTGCC 3167
CTCTCGCTCC CCCACCCAGC CCACCCATTG AAGTCTCCTT GGGTCCCAAA GGTGGGCATG 3227
GTACCGGGGA CTTGGGAGAG TGAGACCCAG TGGAGGGAGC AAGAGGAGAG GGATGTGGGG 3287
GGGTGGGGCA CGGGTAGGGG AAATGGGGTG AACGGTGCTG GCAGTTCGGC TAGATTTCTG 3347
TCTTGTTTGT TTTTTTGTTT TGTTTAATGT ATATTTTTAT TATAATTATT ATATAT 3403
INFORMATION FOR SEQ ID NO: 3
   LENGTH: 7
   TYPE: Amino acid
   TOPOLOGY: Linear
   MOLECULE TYPE: Peptide
   FRAGMENT TYPE: Internal fragment
   SEQUENCE DESCRIPTION: SEQ ID NO:3 
Pro Arg Cys Gly Val Pro Asp
1               5
INFORMATION FOR SEQ ID NO: 4
   LENGTH: 9
   TYPE: Amino acid
   TOPOLOGY: Linear
   MOLECULE TYPE: Peptide
   FRAGMENT TYPE: Internal fragment
   SEQUENCE.DESCRIPTION: SEQ ID NO:4 
Gly Asp Ala His Phe Asp Asp Asp Glu
 1               5
INFORMATION FOR SEQ ID NO: 5
   LENGTH: 20
   TYPE: Nucleic acid
   STRANDEDNESS: Double strand
   TOPOLOGY: Linear
   MOLECULE TYPE: Other nucleic acid, synthetic DNA
   SEQUENCE DESCRIPTION: SEQ ID NO:5 CCMMGVTGYS GVRWBCCWGA 20
INFORMATION FOR SEQ ID NO: 6
   LENGTH: 25
   TYPE: Nucleic acid
   STRANDEDNESS: Double strand
   TOPOLOGY: Linear
   MOLECULE TYPE: Other nucleic acid, synthetic DNA
   SEQUENCE DESCRIPTION: SEQ ID NO:6 YTCRTSVTCR TCRAARTGRR HRTCY 25
INFORMATION FOR SEQ ID NO: 7
   LENGTH: 30
   TYPE: Amino acid
   TOPOLOGY: Linear
   MOLECULE TYPE: Peptide
   FRAGMENT TYPE: Internal fragment
   SEQUENCE DESCRIPTION: SEQ ID NO:7 
Gly Gly Gly Ala Val Ser Ala Ala Ala Val
1               5                   10
Val Leu Pro Val Leu Leu Leu Leu Leu Val
                15                  20
Leu Ala Val Gly Leu Ala Val Phe Phe Phe
                25                  30
INFORMATION FOR SEQ ID NO: 8
   LENGTH: 14
   TYPE: Amino acid
   TOPOLOGY: Linear
   MOLECULE TYPE: Peptide
   SEQUENCE DESCRIPTION: SEQ ID NO:8 
Arg Glu Val Pro Tyr Ala Tyr Ile Arg Glu
 1               5                   10
Gly His Glu Lys
INFORMATION FOR SEQ ID NO: 9
   LENGTH: 14
   TYPE: Amino acid
   TOPOLOGY: Linear
   MOLECULE TYPE: Peptide
   SEQUENCE DESCRIPTION: SEQ ID NO:9 
Asp Gly Asn Phe Asp Thr Val Ala Met Leu
1               5                   10
Arg Gly Glu Met
INFORMATION FOR SEQ ID NO: 10
   LENGTH: 15
   TYPE: Amino acid
   TOPOLOGY: Linear
   MOLECULE TYPE: Peptide
   SEQUENCE DESCRIPTION: SEQ ID NO:10

Pro Lys Ser Ala Leu Arg Asp Trp Met Gly
1               5                   10
Cys Pro Ser Gly Gly
                15

## Claims

1. A human matrix metalloproteinase designated MT-MMP having a length of 582 amino acids, having the amino acid sequences SEQ ID NO:3, SEQ ID NO:8, SEQ ID NO:9 and SEQ ID NO:10 at amino acid numbers 91-97, 160-173, 320-333 and 498-512, respectively, and having the sequence of hydrophobic amino acids shown in SEQ ID NO:7 at amino acid number 533-562.

2. A DNA encoding the polypeptide according to claim 1.

3. Use of the DNA according to claim 2 as a probe.

4. An expression plasmid containing a DNA according to claim 2.

5. A host cell harboring the expression plasmid according to claim 4.

6. A monoclonal antibody which recognizes the polypeptide according to claim 1.

7. The monoclonal antibody according to claim 6 which recognizes the amino acid sequences SEQ ID NO:8, SEQ ID NO:9 or SEQ ID NO:10.

8. The monoclonal antibody 114-2F2 according to claim 6 or 7 which is produced by a hybridoma deposited under FERM BP-4743.

9. A hybridoma producing a monoclonal antibody according to any one of claims 6 to 8.

10. The hybridoma according to claim 9-and deposited under FERM BP-4743, producing the monoclonal antibody 114-2F2.

11. The use of the DNA according to claim 2 or the monoclonal antibody according to any one of claims 6 to 8 for the preparation of a composition for the diagnosis of the presence of tumor cells or the degree of tumor malignancy.

## Patentansprüche

1. Eine menschliche Matrix-Metalloproteinase, bezeichnet mit MT-MMP, mit einer Länge von 582 Aminosäuren, mit den Aminosäuresequenzen SEQ ID NO:3, SEQ ID NO:8, SEQ ID NO:9 und SEQ ID NO:10 mit den Aminosäurepositionen 91-97, 160-173, 320-333 bzw. 498-512, und mit der in SEQ ID NO:7 bei den Aminosäurepositionen 533-562 gezeigten Sequenz hydrophober Aminosäuren.

2. Eine DNA, die das Polypeptid gemäß Anspruch 1 kodiert.

3. Verwendung der DNA gemäß Anspruch 2 als Sondenmolekül.

4. Ein Expressionsplasmid, enthaltend eine DNA gemäß Anspruch 2.

5. Ein Wirtszelle, enthaltend das Expressionsplasmid gemäß Anspruch 4.

6. Ein monoklonaler Antikörper, der das Polypeptid gemäß Anspruch 1 erkennt.

7. Der monoklonale Antikörper nach Anspruch 6, der die Aminosäuresequenzen SEQ ID NO:8, SEQ ID NO:9 oder SEQ ID NO:10 erkennt.

8. Der monoklonale Antikörper 114-2F2 nach Anspruch 6 oder 7, gebildet von einem Hybridom, hinterlegt unter FERM BP-4743.

9. Ein Hybridom, das einen monoklonalen Antikörper nach einem der Ansprüche 6 bis 8 bildet.

10. Das Hybridom nach Anspruch 9, hinterlegt unter FERM BP-4743, das den monoklonalen Antikörper 114-2F2 bildet.

11. Die Verwendung der DNA nach Anspruch 2 oder des monoklonalen Antikörpers nach einem der Ansprüche 6 bis 8 zur Herstellung einer Zusammensetzung zur Diagnose des Vorliegens von Tumorzellen oder dem Ausmaß der Tumormalignität.

## Revendications

1. Métalloprotéase de matrice humaine, dénommée MT-MMP, ayant une longueur de 582 acides aminés, ayant les séquences d'acides aminés SEQ ID NO:3, SEQ ID NO:8, SEQ ID NO:9 et SEQ ID NO:10 au niveau des numéros d'acides aminés 91-97, 160-173, 320-333 et 498-512 respectivement, et ayant la séquence d'acides aminés hydrophobes indiquée dans SEQ ID NO:7 au niveau des acides aminés 533-562.

2. ADN codant pour le polypeptide selon la revendication 1.

3. Utilisation de l'ADN selon la revendication 2 comme sonde.

4. Plasmide d'expression contenant un ADN selon la revendication 2.

5. Cellule hôte hébergeant le plasmide d'expression selon la revendication 4.

6. Anticorps monoclonal qui reconnaît le polypeptide selon la revendication 1.

7. Anticorps monoclonal selon la revendication 6, qui reconnaît les séquences d'acides aminés SEQ ID NO:8, SEQ ID NO:9 ou SEQ ID NO:10.

8. Anticorps monoclonal 114-2F2 selon la revendication 6 ou 7, qui est produit par un hybridome déposé sous la référence FERM BP-4743.

9. Hybridome produisant un anticorps monoclonal selon l'une quelconque des revendications 6 à 8.

10. Hybridome selon la revendication 9 et déposé sous la référence FERM BP-4743, produisant l'anticorps monoclonal 114-2F2.

11. Utilisation de l'ADN selon la revendication 2 ou de l'anticorps monoclonal selon l'une quelconque des revendications 6 à 8 pour la préparation d'une composition pour le diagnostic de la présence de cellules tumorales ou du degré de malignité tumorale.
